**Europäisches Patentamt**

**European Patent Office**

**Office européen des brevets**

(11) Numéro de publication : **0 393 166 B1**

## (12) FASCICULE DE BREVET EUROPEEN

(45) Date de publication du fascicule du brevet :
26.08.92 Bulletin 92/35

(21) Numéro de dépôt : **89908199.6**

(22) Date de dépôt : **30.06.89**

(86) Numéro de dépôt international :
**PCT/FR89/00342**

(87) Numéro de publication internationale :
**WO 90/00073 11.01.90 Gazette 90/02**

(51) Int. Cl.⁵ : **A61M 5/32**

(54) SERINGUE DU TYPE COMPORTANT UNE AIGUILLE D'INJECTION A USAGE UNIQUE, NOTAMMENT POUR LE DOMAINE DENTAIRE.

(30) Priorité : **30.06.88 FR 8808987**

(43) Date de publication de la demande :
**24.10.90 Bulletin 90/43**

(45) Mention de la délivrance du brevet :
**26.08.92 Bulletin 92/35**

(84) Etats contractants désignés :
**AT BE CH DE FR GB IT LI LU NL SE**

(56) Documents cités :
**EP-A- 0 288 003**
**DE-A- 1 566 563**
**US-A- 2 571 653**

(56) Documents cités :
**US-A- 2 925 083**
**US-A- 4 702 738**
**US-A- 4 747 829**
**US-A- 4 752 290**

(73) Titulaire : **BRUNEL, Marc**
**Saint-Médard**
**F-32300 Mirande (FR)**

(72) Inventeur : **BRUNEL, Marc**
**Saint-Médard**
**F-32300 Mirande (FR)**

(74) Mandataire : **Morelle, Guy Georges Alain et al**
**CABINET MORELLE & BARDOU, SC 5,**
**Boulevard de la Méditerranée**
**F-31400 Toulouse (FR)**

## Description

L'invention concerne une seringue du type comportant une aiguille d'injection à usage unique, notamment pour le domaine dentaire.

Les seringues classiques utilisées dans le domaine dentaire comportent une poignée dotée d'un piston et d'ailettes de prises digitales et un corps de seringue apte à être vissé de façon amovible sur cette poignée après la mise en place d'une cartouche. Ce corps comporte, en outre, un nez fileté permettant le vissage d'une embase portant une aiguille, conditionnée initialement dans un étui protecteur de façon à éviter tout risque de piqûre lors de la manipulation de cette aiguille et du vissage de l'embase sur le corps de seringue. L'étui retiré en vue de l'utilisation de la seringue est ensuite remis en place en vue du dévissage de l'embase. Ce type de seringue présente quatre inconvénients majeurs. en premier lieu, lors du recapuchonnage de l'aiguille, l'étui est présenté en regard de l'extrémité de cette dernière et les accidents par piqûre sont relativement fréquents avec tous les risques que comporte une piqûre avec une aiguille souillée. En outre, le corps de seringue destiné à être réutilisé est souillé par la salive du patient, ce qui impose une stérilisation complète de ce corps après usage. Par ailleurs, la cartouche n'est pas protégée lors de l'injection en bouche, malgré les risques d'éclatement accidentel de cette dernière, dûs aux pressions élevées nécessaires pour l'injection du liquide. Enfin, les opérations de vissage et dévissage, d'une part de l'embase sur le corps de seringue et, d'autre part, du corps de seringue sur la poignée, constituent des opérations nécessitant un temps relativement long au praticien.

Dans un autre domaine que le domaine dentaire, il existe également des seringue conçues de façon à pallier certains des inconvénients ci-dessus évoqués. Ces seringues, telles que décrites dans le brevet US 4.702.738, comportent un corps de seringue et une poignée d'un seul tenant et un étui protecteur apte à coulisser extérieurement le long du corps de seringue entre une position arrière où l'aiguille est dégagée et une position avant où cet étui recouvre totalement l'aiguille. Ces seringues pallient donc les trois premiers inconvénients ci-dessus évoqués des seringues dentaires classiques. Par contre, de par leur conception, elles nécessitent de jeter la totalité de la seringue après usage. De plus, une telle seringue modifiée pour l'adapter au domaine dentaire ne résoud pas les problèmes posés par les temps de montage et de démontage des divers éléments.

La présente invention vise à pallier les inconvénients des seringues connues et a pour objectif de fournir une seringue comportant une aiguille d'injection à usage unique, de conception très simple, éliminant tout risque de piqûre avec cette aiguille, avant et après utilisation, et dont le montage et le démontage nécessitent des opérations très rapides et aisées.

Un autre objectif est de fournir une seringue dont le corps peut être conditionné sous emballage stérile.

Un autre objectif est de fournir une seringue protégeant le patient contre tout risque d'éclatement de la cartouche, tout en permettant de visualiser le niveau de liquide dans cette cartouche.

Un autre objectif est de fournir une seringue interdisant toute réutilisation ultérieure de la cartouche.

A cet effet, l'invention vise une seringue du type comportant une aiguille d'injection à usage unique, notamment pour le domaine dentaire, et comprenant :
- une poignée de seringue comportant un piston et une pièce d'appui traversée par ledit piston et mobile le long de celui-ci,
- un corps de seringue doté vers une de ses extrémités d'une embase portant l'aiguille d'injection de façon à délimiter une partie active d'injection,
- des moyens de fixation amovible du corps de seringue sur la poignée de seringue, et
- un étui protecteur de forme adaptée pour loger l'aiguille et son embase.

Selon cette invention, cette seringue se caractérise en ce que les moyens de fixation amovible du corps de seringue sur la poignée comprennent :
- un embout tubulaire porté par le corps de seringue à l'opposé de l'embase, et doté d'une structure d'emboîtement,
- un manchon solidaire de la pièce d'appui de la poignée de façon à être traversé par le piston, et doté d'une structure d'emboitement conjuguée de celle de l'embout du corps de seringue,
- lesdits embout tubulaire et manchon étant de formes adaptées pour pouvoir pénétrer l'un dans l'autre de façon à venir dans une position emboîtée où leurs structures d'emboîtement coopèrent,
- l'étui protecteur présente un diamètre adapté pour pouvoir coulisser extérieurement le long du corps de seringue, avec un tronçon de verrouillage apte à coiffer l'embout tubulaire et ledit manchon, et à les verrouiller dans leur position emboîtée,
- l'étui protecteur et le corps de seringue sont dotés de moyens de butée adaptés pour définir deux positions extrêmes : une position dite avant où l'étui recouvre totalement l'aiguille et l'embase, et une position dite arrière où au moins la partie active de l'aiguille est dégagée et où le tronçon de verrouillage de l'étui coiffe l'embout tubulaire et ledit manchon emboîtés de façon à assurer un verrouillage de la liaison corps de seringue/poignée de seringue.

Les manipulations requises pour le montage et le démontage de cette seringue sont notablement simplifiées par rapport aux seringues dentaires connues. En effet, l'assemblage du corps de seringue et de la poignée ne nécessite qu'un simple emboîtement, verrouillé par la suite lors du coulissement de l'étui protecteur vers l'arrière. Cet étui joue donc un double rôle puisque, d'une part, il assure la protection du praticien contre tout risque de piqûre avant et après utilisation et, d'autre part, il permet de réaliser un assemblage rapide du corps de seringue sur la poignée, exempt de tout risque de désassemblage accidentel.

Au niveau protection, l'étui protège dans un premier temps l'aiguille lors de l'assemblage du corps de seringue sur la poignée. En outre, lorsque l'étui est amené à coulisser vers l'avant, une fois l'injection en bouche effectuée, les risques de piqûre sont nuls car l'aiguille est recouverte en déplaçant cet étui du corps de seringue vers l'extrémité de l'aiguille. L'ensemble corps de seringue-étui protecteur peut alors être jeté, l'aiguille étant à nouveau logée dans cet étui protecteur. On constate donc que le praticien n'encourt aucun risque de piqûre quelle que soit l'opération effectuée.

Au niveau verrouillage, l'étui protecteur vient enserrer l'embout tubulaire et le manchon emboîtés du corps de seringue et de la poignée et permet donc de transformer ce simple emboîtement en un assemblage rigide exempt de tout risque de désassemblage accidentel. Il est à noter, en outre, que lorsque l'étui est ramené vers sa position avant, le corps de seringue et la poignée se désemboîtent automatiquement sous l'effet de la traction imprimée à cet étui. De plus, du fait que l'étui assure le verrouillage de l'emboîtement, le corps de seringue ne peut être démonté et jeté sans que l'aiguille soit protégée par cet étui.

Par ailleurs, le corps de seringue peut comporter un nez fileté apte à permettre le vissage d'une embase dotée d'un alésage taraudé. Toutefois, cette embase est préférentiellement scellée sur l'extrémité du corps de seringue. En effet, l'aiguille étant initialement scellée sur son embase, elle-même scellée sur l'extrémité du corps de seringue, les manipulations nécessaires au vissage de cette aiguille sont ainsi supprimées.

De plus, le corps de seringue et l'étui protecteur sont préférentiellement réalisés en un matériau plastique transparent. De ce fait, le praticien peut visualiser le niveau de liquide restant dans la cartouche.

Selon un mode de réalisation préférentiel, les structures d'emboîtement de l'embout et du manchon du corps,et de la poignée de seringue sont constituées d'une nervure annulaire et d'une gorge annulaire de formes conjuguées, ménagées de façon à venir coopérer dans la position emboîtée desdits embout et manchon.

Outre sa simplicité, ce système d'emboîtement permet, tout en bloquant en translation le corps de seringue et la poignée, de conférer à cet assemblage un degré de liberté en rotation. Cette disposition permet au praticien d'orienter très facilement l'aiguille de façon à disposer le biseau de cette dernière de façon appropriée lors de l'injection.

Par ailleurs, les moyens de butée sont préférentiellement constitués de deux gorges annulaires externes, dites de butée avant et arrière, ménagées sur le corps de seringue et d'une nervure annulaire interne ménagée dans l'étui protecteur.

En outre, selon une autre caractéristique de l'invention, la seringue comporte une gorge annulaire de blocage ménagée sur le corps de seringue entre l'embase et la gorge de butée avant, ladite gorge de blocage présentant une section rectangulaire conjuguée de celle de la nervure de l'étui protecteur et étant adaptée pour interdire tout déplacement dudit étui lorsque ladite nervure est logée à l'intérieur de cette gorge de blocage.

Cette gorge de blocage, dans laquelle vient se loger la nervure de l'étui protecteur lorsque ce dernier est amené à coulisser vers l'avant, garantit contre toute ré-utilisation ultérieure du corps de seringue ou contre toute piqûre accidentelle lorsque ce corps de seringue est manipulé après avoir été jeté.

L'invention s'étend en tant qu'élément essentiel constitutif de cette seringue, en premier lieu à un ensemble d'injection à usage unique comprenant :

– un corps de seringue doté, vers une de ses extrémités, d'une embase portant une aiguille d'injection de façon à délimiter une partie active d'injection, et vers son extrémité opposée, d'un embout tubulaire comportant une structure d'emboîtement,

– un étui protecteur de diamètre adapté pour pouvoir coulisser extérieurement le long du corps de seringue, avec un tronçon de verrouillage apte à coiffer l'embout tubulaire dudit corps de seringue,

– des moyens de butée ménagés sur l'étui protecteur et le corps de seringue et adaptés pour définir deux positions extrêmes : une position dite avant, où l'étui recouvre totalement l'aiguille et l'embase et, une position dite arrière, où au moins la partie active de l'aiguille est dégagée et où le tronçon de verrouillage de l'étui coiffe l'embout tubulaire du corps de seringue.

Cette invention s'étend aussi, en tant qu'élément essentiel constitutif de la seringue à une poignée de seringue comprenant :

– une pièce d'appui digital comportant un manchon doté d'une structure d'emboîtement,

– un piston mobile à l'intérieur de la pièce d'appui du manchon.

L'invention s'étend également à un module consommable à usage unique pour le domaine dentaire comprenant en combinaison :

– un ensemble d'injection tel que décrit ci-dessus, comportant un corps de seringue, et un étui protecteur disposé dans sa position avant où il recouvre totalement l'aiguille et l'embase,

– une cartouche intégrée à l'intérieur du corps de seringue,

– une pochette stérilisée de conditionnement dudit ensemble d'injection.

L'invention décrite ci-dessus de façon générale sera mieux comprise à la lecture de la description détaillée qui suit en référence aux dessins annexés qui en représentent, à titre d'exemple non limitatif, un mode de réalisation préférentiel. Sur ces dessins qui font partie intégrante de la présente description :

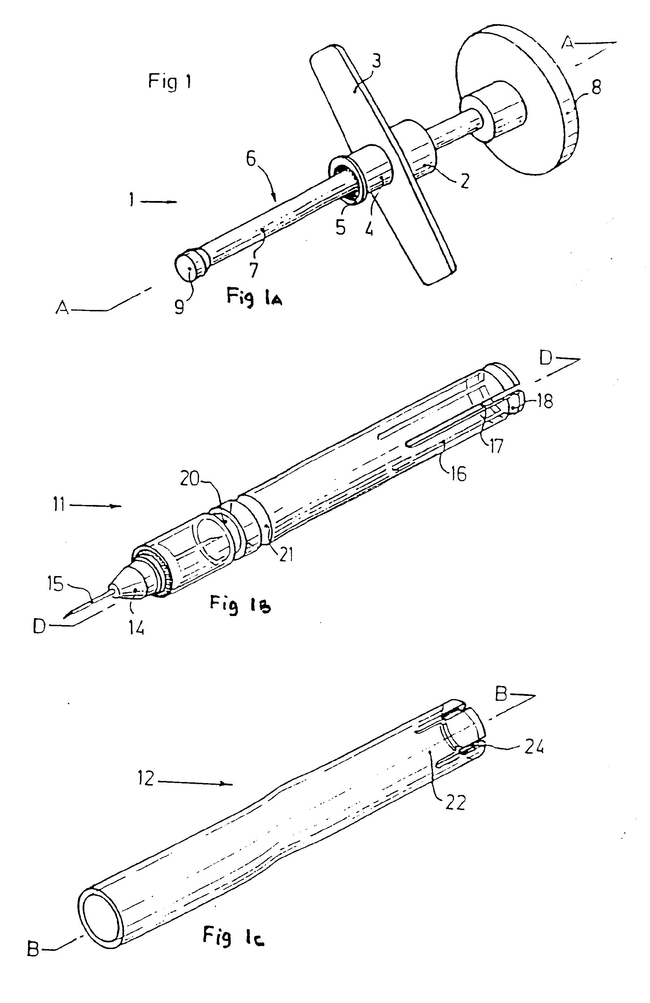

– la Figure 1 est une vue en perspective éclatée des trois éléments, étui protecteur, corps de seringue et poignée, constitutifs d'une seringue conforme à l'invention,

– la Figure 2 est une vue en perspective de l'ensemble étui protecteur/corps de seringue et de la poignée, avant assemblage de ces derniers,

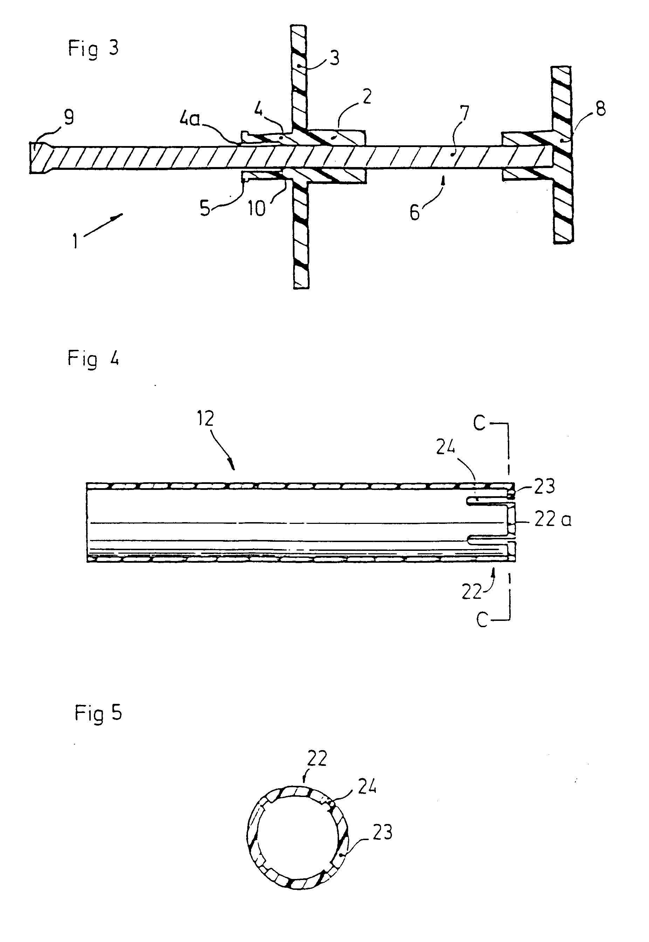

– la Figure 3 est une coupe longitudinale par un plan AA de la poignée d'une seringue conforme à l'invention,

– la Figure 4 est une coupe longitudinale par un plan BB de l'étui protecteur d'une seringue conforme à l'invention,

– la Figure 5 est une coupe transversale par un plan CC de cet étui protecteur,

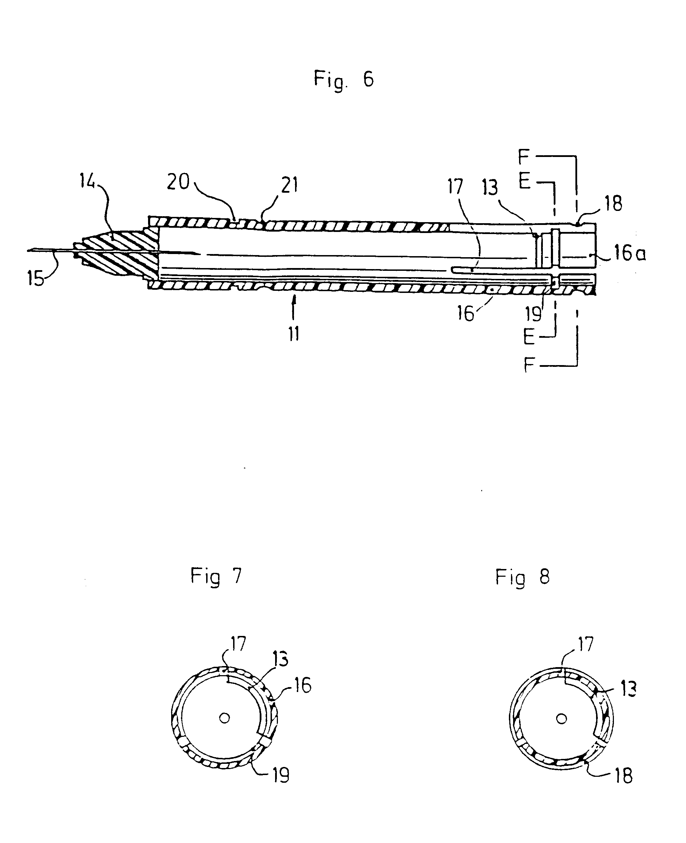

– la Figure 6 est une coupe longitudinale pair un plan DD du corps d'une seringue conforme à l'invention,

– la Figure 7 est une coupe transversale par un plan EE de ce corps de seringue,

– la Figure 8 en est une coupe transversale par un plan FF,

– les Figures 9, 10, 11 sont des coupes longitudinales de cette seringue, respectivement avant l'injection, pendant l'injection et après l'injection.

– la Figure 12 est une vue en perspective d'un module consommable à usage unique pour une seringue conforme à l'invention.

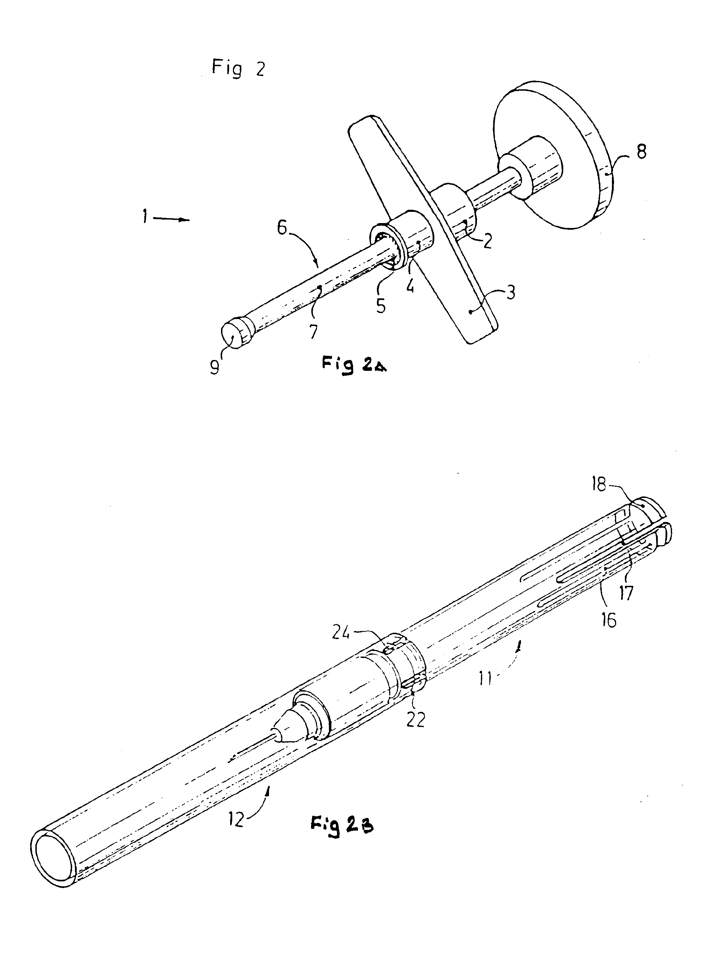

La seringue représentée aux Figures est constituée de trois éléments distincts : un corps de seringue, un étui protecteur et une poignée. L'étui protecteur et le corps de seringue sont, en outre, adaptés pour être assemblés, tel que représenté à la Figure 2, de façon à constituer un ensemble d'injection jetable.

En premier lieu, la poignée 1 de cette seringue comporte une pièce d'appui tubulaire 2 portant des ailettes de prise digitale 3, et possédant, vers une de ses extrémités, un manchon 4 débouchant par une face ouverte 4a. Ce manchon 4 comporte, en outre, au niveau de sa face ouverte 4a une nervure annulaire 5 ménagée sur sa face périphérique externe.

A l'intérieur de la pièce d'appui 2, est disposé un piston manoeuvrable 6 adapté pour pouvoir coulisser à l'intérieur de cette pièce d'appui et du manchon 4. Ce piston 6 comporte une tige 7 de diamètre sensiblement inférieur au diamètre interne de la pièce d'appui 2. Ladite tige est munie à l'une de ses extrémités d'un bouton d'actionnement 8 qui permet de déplacer le piston 6 à l'intérieur de la pièce d'appui 2 de la poignée 1. Elle est munie à son extrémité opposée d'une collerette 9 de diamètre supérieur au diamètre de cette tige.

L'inamovibilité de ce piston 6 par rapport à la pièce d'appui 2 est obtenue au moyen d'un épaulement interne 10 ménagé dans ladite pièce d'appui et délimitant deux tronçons de diamètre interne différents : un tronçon de diamètre interne conjugué de celui de la collerette 9, débouchant au niveau de la face ouverte 4a du manchon 4 et un tronçon de diamètre conjugué de celui de la tige 7.

Le deuxième ensemble de cette seringue est composé d'un corps de seringue 11 doté d'un étui protecteur 12 adapté pour coulisser le long de ce corps de seringue 11.

Ce corps de seringue 11 et cet étui protecteur 12 sont réalisés en un matériau plastique transparent permettant de visualiser le niveau de la cartouche.

Le corps de seringue 11, de forme générale cylindrique, est muni à l'une de ses extrémités d'une embase 14 scellée portant une aiguille d'injection 15, de façon à délimiter une partie active d'injection. Ce corps de seringue 11 possède, en outre, à son extrémité opposée, un embout tubulaire 16 débouchant par une face ouverte 16a et de diamètre interne adapté pour loger le manchon 4 de la poignée 1.

Cet embout tubulaire 16 comporte une pluralité de fentes longitudinales 17 (en exemple 3) s'étendant depuis sa face ouverte 16a, de façon à permettre sa dilatation radiale lorsque cet embout 16 vient au contact de la nervure 5 du manchon 4 de la poignée 1.

Cet embout tubulaire 16 comporte, en outre, une gorge annulaire 18 de forme évasée ménagée sur sa face périphérique externe à distance de son extrémité ouverte 16a. Il comporte également une gorge annulaire interne 19 ménagée de façon à coopérer avec la nervure 5 du manchon 4 de la poignée 1, lorsque ce dernier est logé à l'intérieur de cet embout 16

De plus, ce corps de seringue 11 comporte deux autres gorges annulaires 20, 21 ménagées sur sa face périphérique entre l'embout 16 et l'embase 14 : une gorge annulaire de blocage 20 de section rectangulaire, ménagée à une distance déterminée de l'embase 14 et une gorge annulaire 21 de section évasée ménagée entre la gorge de blocage 20 ci-dessus citée et l'embout tubulaire 16.

En dernier lieu, ce corps de seringue 11 comprend une nervure interne 13 ménagée à l'intérieur de l'embout tubulaire 16, côté embase 14 par rapport à la gorge annulaire 19 dudit embout, ladite nervure présentant une forme dissymétrique adaptée pour permettre l'introduction d'une cartouche 26 à l'intérieur du corps de seringue 11 et interdire le retrait de cette cartouche.

L'étui protecteur 12 présente quant à lui une forme cylindrique et possède un diamètre interne correspondant au diamètre externe du corps de seringue 11.

Cet étui protecteur 12 comporte un tronçon arrière de verrouillage 22 débouchant par une face ouverte 22a et comportant, au niveau de cette face ouverte 22a, une nervure annulaire interne 23 de forme adaptée pour se loger dans les gorges annulaires externes 18, 20, 21 du corps de seringue 11.

Ce tronçon arrière de verrouillage 22 comporte enfin, une pluralité de fentes longitudinales 24 (en exemple 4) s'étendant depuis sa face ouverte 22a et adaptées pour permettre sa dilatation radiale lors du coulissement de la nervure 23 le long du corps de seringue 11.

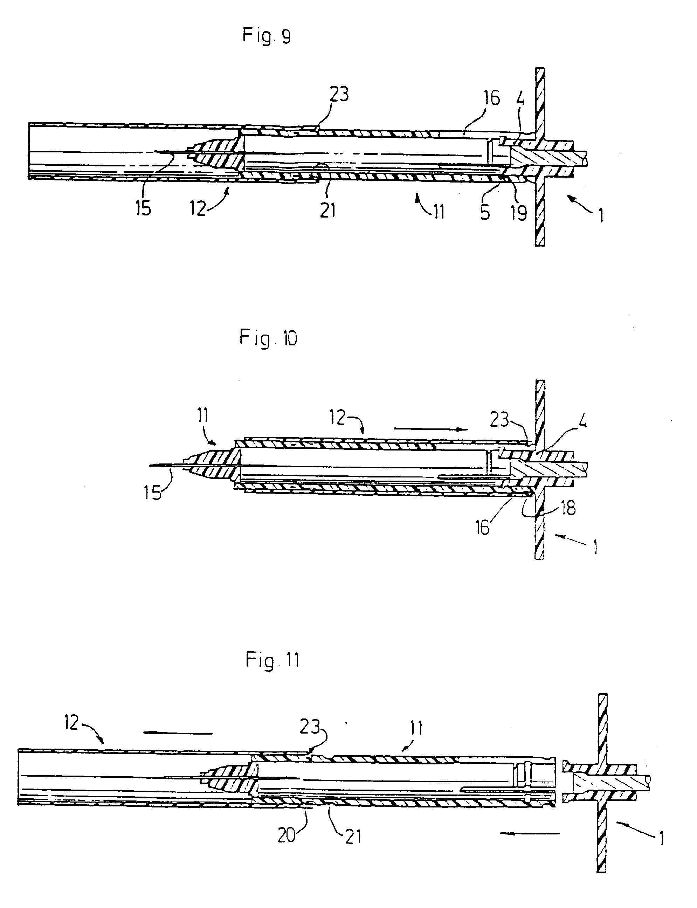

L'utilisation de cette seringue est explicitée ci-dessous en référence aux Figures 9 à 12.

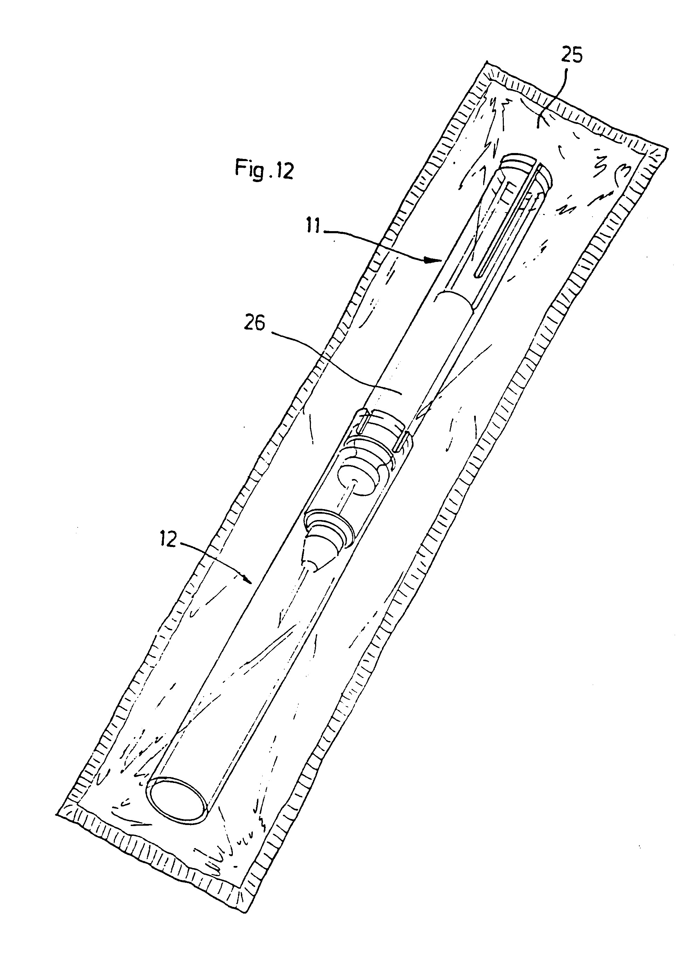

Tel que représenté à la Figure 12, l'ensemble corps de seringue 11/étui protecteur 12 est préalablement conditionné dans une pochette stérile 25, dans une position où la nervure interne 23 de l'étui est logée dans la gorge évasée avant 21 du corps de seringue 11. Dans cette position, l'étui protecteur 12 recouvre totalement l'aiguille 15 et garantit contre tout risque de piqûre ou de détérioration de cette aiguille.

De plus, préalablement au conditionnement, une cartouche 26 est introduite dans le corps de seringue 11 dans lequel elle est maintenue grâce à la nervure interne 13. Il est à noter que cette introduction est notamment facilitée par la présence des fentes 17 qui permettent une dilatation radiale de l'embout tubulaire 16. On obtient ainsi un module consommable prêt à l'emploi.

Une fois l'ensemble d'injection retiré de sa pochette 25, le manchon 4 de la poignée 1 est inséré dans l'embout tubulaire 16 du corps de seringue, dans lequel il vient s'emboîter grâce à la coopération de la nervure annulaire externe 5 et de la gorge annulaire interne 19 (Figure 9).

L'étui protecteur 12 est alors amené à coulisser vers l'arrière jusqu'à ce que sa nervure annulaire interne 23 vienne se loger dans la gorge annulaire 18 de l'embout annulaire 16 du corps de seringue 11, position dans laquelle la partie active de l'aiguille 15 est dégagée (Figure 10).

En outre, dans cette position, l'étui protecteur 12 coiffe l'embout tubulaire 16 et le manchon 4 de la poignée 1 et du corps de seringue 11 et assure donc le verrouillage de l'emboîtement.

Par ailleurs, lors de l'injection, le praticien peut visualiser le niveau de liquide restant dans la cartouche 26 sans que le patient soit soumis à des risques d'éclatement de cette dernière.

En fin d'injection, l'étui protecteur 12 est amené à coulisser vers l'avant jusqu'à ce que sa nervure interne 23 vienne se loger dans la gorge de blocage 20 du corps de seringue 11, après être passée dans la gorge annulaire 21 de forme évasée. La traction effectuée sur cet étui protecteur 12 pour amener son coulissement provoque, en outre, automatiquement, le désemboîtement du corps de seringue 11 et de la poignée 1.

L'ensemble corps de seringue 11/étui protecteur 12 peut alors être jeté, sans risque de ré-utilisation ultérieure ou de piqûre accidentelle si cet ensemble est manipulé après avoir été jeté.

De plus la forme de la nervure 13 interdit de retirer la cartouche 26 et garantit contre toute ré-utilisation éventuelle de cette dernière.

**Revendications**

1. Seringue comportant une aiguille d'injection (15) à usage unique, notamment pour le domaine dentaire et comprenant :
   – une poignée (1) de seringue comportant un piston (6) et une pièce d'appui (2, 3) traversée par ledit piston et mobile le long de celui-ci,
   – un corps (11) de seringue doté vers une de ses extrémités d'une embase (14) portant l'aiguille d'injection (15) de façon à délimiter une partie active d'injection,
   – des moyens de fixation amovible (4, 5, 16, 19) du corps de seringue (11) sur la poignée de seringue (1),
   – et un étui protecteur (12) de forme adaptée pour loger l'aiguille (15) et son embase (14), ladite seringue étant CARACTERISEE EN CE QUE :
   – les moyens de fixation amovible du corps de seringue sur la poignée comprennent :
   – un embout tubulaire (16) porté par le corps de seringue (11) à l'opposé de l'embase (14), et doté d'une structure d'emboîtement (19),
   – un manchon (4) solidaire de la pièce d'appui (2, 3) de la poignée (1) de façon à être traversé par le piston (6), et doté d'une structure d'emboîtement (5) conjuguée de celle de l'embout (16) du corps de seringue (11), lesdits embout tubulaire (16) et manchon (4) étant de formes adaptées pour pouvoir pénétrer l'un dans l'autre de façon à venir dans une position emboîtée où leurs structures d'emboîtement (5, 19) coopèrent,
   – l'étui protecteur (12) présente un diamètre adapté pour pouvoir coulisser extérieurement le long du corps de seringue (11), avec un tronçon de verrouillage (22) apte à coiffer l'embout tubulaire (16) et ledit manchon (4), et à les verrouiller dans leur position emboîtée,
   – l'étui protecteur (12) et le corps de seringue (11) sont dotés de moyens de butée (18, 21, 23) adaptés pour définir deux position extrêmes : une position dite avant où l'étui (12) recouvre totalement l'aiguille (15) et l'embase (14), et une position dite arrière où au moins la partie active de l'aiguille (15) est dégagée et où le tronçon de verrouillage (22) de l'étui (12) coiffe l'embout tubulaire (16) et ledit manchon (4) emboîtés, de façon à assurer un verrouillage de la liaison corps de seringue/poignée de seringue.

2. Seringue selon la Revendication 1, CARACTERISEE EN CE QUE les structures d'emboîtement de l'embout (16) et du manchon (4) du corps (11) et de la poignée (1) de seringue sont constituées d'une nervure annulaire (5) et d'une gorge annulaire (19) de formes conjuguées, ménagées de façon à venir coopérer dans la position emboîtée desdits embout et manchon.

3. Seringue selon la Revendication 2, CARACTERISEE EN CE QUE le manchon (4) de la poignée de seringue (1) est de forme adaptée pour pénétrer dans l'embout (16) du corps de seringue (11),
   – la nervure annulaire (5) est ménagée sur la manchon (4) de la poignée de seringue (1), vers l'extrémité de ce dernier,
   – la gorge annulaire (19) est ménagée à l'intérieur de l'embout (16) du corps de seringue (11), à distance de l'extrémité de ce dernier, de façon à obtenir une position emboîtée dans laquelle le manchon (4) de la poignée (1) est logé dans l'embout (16) du corps de seringue (11).

4. Seringue selon l'une des Revendications 2 ou 3, CARACTERISEE EN CE QUE l'embout (16) du corps de seringue (11) comprend une pluralité de fentes longitudinales (17) s'étendant depuis son extrémité (16a) de façon à permettre une déformation radiale dudit embout.

5. Seringue selon l'une des Revendications précédentes, CARACTERISEE EN CE QUE les moyens de butée de l'étui protecteur (12) sont constitués de deux gorges annulaires externes (21, 18) dites de butée avant et arrière, ménagées sur le corps de seringue (11), et d'une nervure annulaire interne (23) ménagée dans l'étui protecteur (12).

6. Seringue selon la Revendication 5, CARACTERISEE EN CE QUE la nervure annulaire (23) de l'étui protecteur (12) est ménagée vers l'extrémité du tronçon de verrouillage (22) de ce dernier,
   – la gorge de butée arrière (18) est ménagée vers l'extrémité de l'embout (16) du corps de seringue (11) de façon à obtenir une position de butée arrière de l'étui protecteur (12) dans laquelle ce dernier coiffe l'embout tubulaire (16) et le manchon (4) emboîtés,
   – et la gorge de butée avant (21) est ménagée sur le corps de seringue (11) à une distance de l'embase (14) adaptée pour obtenir une position de butée avant de l'étui protecteur (12) dans laquelle ce dernier recouvre totalement l'aiguille (15) et ladite embase (14).

7. Seringue selon l'une des Revendication 5 ou 6, CARACTERISEE EN CE QUE l'étui protecteur (12) comporte un tronçon de verrouillage (22) doté d'une pluralité de fentes longitudinales (24) s'étendant depuis son extrémité de façon à permettre une déformation radiale dudit tronçon.

8. Seringue selon la Revendication 6, CARACTERISEE EN CE QU'elle comporte une gorge annulaire de blocage (20) ménagée sur le corps de seringue (11) entre l'embase (14) et la gorge de butée avant (21), ladite gorge de blocage présentant une section rectangulaire conjuguée de celle de la nervure (23) de l'étui protecteur (12) et étant adaptée pour interdire tout déplacement dudit étui lorsque ladite nervure (23) est logée à l'intérieur de cette gorge de blocage (20).

9. Seringue selon la Revendication 3, CARACTERISEE EN CE QU'elle comprend une nervure interne (13) ménagée à l'intérieur de l'embout tubulaire (16) du corps de seringue (11), côté embase (14) par rapport à la gorge annulaire (19) dudit embout, ladite nervure présentant une forme dissymétrique adaptée pour permettre l'introduction d'une cartouche (26) à l'intérieur du corps de seringue (11) et interdire le retrait de cette cartouche.

10. Seringue selon la Revendication 9, CARACTERISEE EN CE QU'elle comprend un corps de seringue (11) et un étui protecteur (12) réalisés en un matériau plastique transparent adaptée pour permettre de visualiser le niveau de liquide contenu dans la cartouche (26).

11. Seringue selon l'une de Revendications précédentes, CARACTERISEE EN CE QU'elle comprend une embase (14) scellée sur l'extrémité du corps de seringue (11).

12. Seringue selon l'une des Revendications 1 à 10, CARACTERISEE EN CE QUE le corps de seringue comporte un nez fileté apte à permettre le vissage d'une embase dotée d'un alésage taraudé.

13. Ensemble d'injection à usage unique pour une seringue conforme à l'une des Revendications 1 à 12, CARACTERISE EN CE QU'il comprend :
    – un corps de seringue (11) doté, vers une de ses extrémités, d'une embase (14) portant une aiguille d'injection (15) de façon à délimiter une partie active d'injection, et vers son extrémité opposée, d'un embout tubulaire (16) comportant une structure d'emboîtement (19),
– un étui protecteur (12) de diamètre adapté pour pouvoir coulisser extérieurement le long du corps de seringue (11), avec un tronçon de verrouillage (22) apte à coiffer l'embout tubulaire (16) dudit corps de seringue,
– des moyens de butée ménagés sur l'étui protecteur (12) et le corps de seringue (11), adaptés pour définir deux positions extrêmes : une position dite avant, où l'étui (12) recouvre totalement l'aiguille (14) et l'embase (15), et une position dite arrière, où au moins la partie active de l'aiguille (15) est dégagée et où le tronçon de verrouillage (22) de l'étui (12) coiffe l'embout tubulaire (16) du corps de seringue (11).

14. Ensemble d'injection selon la Revendication 13, CARACTERISEE EN CE QUE la structure d'emboîtement de l'embout tubulaire (16) du corps de seringue (11) est constituée d'une gorge annulaire (19).

15. Ensemble d'injection selon l'une des Revendications 13 ou 14, CARACTERISE EN CE QUE l'embout tubulaire (16) au corps de seringue (11) comprend une pluralité de fentes longitudinales (17) s'étendant depuis son extrémité.

16. Ensemble d'injection selon l'une des Revendications 13 à 15, CARACTERISE EN CE QUE les moyens de butée de l'étui protecteur (12) sont constitués de deux gorges annulaires externes (21, 18) dites de butée avant et arrière, ménagées sur le corps de seringue (11), et d'une nervure annulaire interne (23) ménagée dans l'étui protecteur (12).

17. Ensemble d'injection selon la Revendication 16, CARACTERISE EN CE QUE :
– la nervure annulaire (23) de l'étui protecteur (12) est ménagée vers l'extrémité du tronçon de verrouillage (22) de ce dernier,
– la gorge de butée arrière (18) est ménagée vers l'extrémité de l'embout (16) du corps de seringue (11) de façon à obtenir une position de butée arrière de l'étui protecteur (12) dans laquelle ce dernier coiffe l'embout tubulaire (16) du corps de seringue (11),
– et la gorge de butée avant (21) est ménagée sur le corps de seringue (11) à une distance de l'embase (14) adaptée pour obtenir une position de butée avant de l'étui protecteur (12) dans laquelle ce dernier recouvre totalement l'aiguille (15) et ladite embase (14).

18. Ensemble d'injection selon l'une des Revendications 16 ou 17, CARACTERISEE EN CE QUE l'étui protecteur (12) comporte un tronçon de verrouillage (22) doté d'une pluralité de fentes longitudinales (24) s'étendant depuis son extrémité de façon à permettre une déformation radiale dudit tronçon.

19. Ensemble d'injection selon la Revendication 17, CARACTERISE EN CE QUE le corps de seringue (11) comporte une gorge annulaire de blocage (20) ménagée entre l'embase (14) et la gorge de butée avant (21), ladite gorge de blocage présentant une section rectangulaire conjuguée de celle de la nervure (23) de l'étui protecteur (12) et étant adaptée pour interdire tout déplacement dudit étui lorsque ladite nervure (23) est logée à l'intérieur de cette gorge de blocage (20).

20. Ensemble d'injection selon la Revendication 14, CARACTERISE EN CE QUE le corps de seringue (11) comprend une nervure interne (13) ménagée à l'intérieur de l'embout tubulaire (16), côté embase (14) par rapport à la gorge annulaire (19) dudit embout, ladite nervure présentant une forme dissymétrique adaptée pour permettre l'introduction d'une cartouche (26) à l'intérieur du corps de seringue (11) et interdire le retrait de cette cartouche.

21. Ensemble d'injection selon la Revendication 20, CARACTERISE EN CE QU'il comprend un corps de seringue (11) et un étui protecteur (12) réalisés en un matériau plastique transparent adapté pour permettre de visualiser le niveau de liquide contenu dans la cartouche (26).

22. Ensemble d'injection selon l'une des Revendications 13 à 21, CARACTERISE EN CE QU'il comprend une embase (14) scellée sur l'extrémité du corps de seringue (11).

23. Ensemble d'injection selon l'une des Revendications 13 à 21, dans lequel le corps de seringue comporte un nez fileté apte à permettre le vissage d'une embase dotée d'un alésage taraudé.

24. Module consommable à usage unique, pour le domaine dentaire, CARACTERISE EN CE QU'il comprend en combinaison :
– un ensemble d'injection conforme à l'une des Revendications 13 à 23, comportant un corps de seringue (11), et un étui protecteur (12) disposé dans sa position avant où il recouvre l'aiguille (15) et l'embase (14),

– une cartouche (26) intégrée à l'intérieur du corps de seringue (11),
– une pochette stérilisée (25) de conditionnement dudit ensemble d'injection.

25. Module consommable selon la Revendication 24, CARACTERISE EN CE QU'il comprend un ensemble d'injection conforme à la Revendication 20.

26. Poignée pour seringue conforme à l'une des Revendications 1 à 12, CARACTERISEE EN CE QU'elle comprend :
    – une pièce d'appui digital (2, 3) comportant un manchon (4) doté d'une structure d'emboîtement (5),
    – un piston (6) mobile à l'intérieur de la pièce d'appui (2, 3) et du manchon (4).

27. Poignée selon la Revendication 26, CARACTERISEE EN CE QUE la structure d'emboîtement du manchon (4) est constituée d'une nervure annulaire (5) ménagée au niveau de l'extrémité dudit manchon.

**Patentansprüche**

1. Spritze mit Injektionsnadel (15) zur Einmalverwendung, insbesondere für Zahnärzte mit
    a) einem Spritzengriff (1), der einen Kolben (6) und ein Stützteil (2, 3) aufweist, durch welches der Kolben verläuft und welches längs dieses Kolbens beweglich ist,
    b) einem Spritzenkörper (11), der an einem seiner Enden einen Ansatz (14) aufweist, welcher die Injektionsnadel (15) derart trägt, daß ein aktiver Injektionsteil begrenzt wird,
    c) Mitteln (4, 5, 16, 19), um den Spritzenkörper (11) lösbar auf dem Spritzengriff (1) zu befestigen sowie
    d) einer Schutzhülle (12), die derart gestaltet ist, daß sie die Nadel (15) und ihren Ansatz (14) aufnehmen kann,
    dadurch gekennzeichnet, daß
    e) die Mittel, um den Spritzenkörper abnehmbar auf dem Griff zu befestigen,
        1) eine rohrförmige Hülse (16) aufweisen, welche vom Spritzenkörper an dem dem Ansatz (14) entgegengesetzten Ende getragen und mit einer Einsetz-Vorrichtung (19) versehen ist, und
        2) eine Muffe (4) aufweisen, die einteilig mit dem Stützteil (2, 3) des Griffes (1) ausgebildet, so daß sie vom Kolben (6) durchquert wird, und mit einer Einsetzvorrichtung (5) versehen ist, welche der (19) der Hülse (16) des Spritzenkörpers (11) entspricht, wobei die rohrförmige Hülse (16) und die Muffe (4) in ihrer Gestalt derart aneinander angepaßt sind, daß sie ineinander eindringen können, um in eine Einsetzstellung zu gelangen, wo ihre Einsetzvorrichtungen (5, 19) zusammenarbeiten,
    f) die Schutzhülle (12) einen derartigen Durchmesser aufweist, daß sie außen längs des Spritzenkörpers (11) gleiten kann, und mit einem hinteren Verriegelungsabschnitt (22) versehen ist, der die rohrförmige Hülse (16) und die genannte Muffe (4) übergreifen und diese miteinander in ihrer Einsetzstellung verriegeln kann,
    g) die Schutzhülle (12) und der Spritzenkörper (11) mit Anschlage mitteln (18, 21, 23) versehen sind, welche zwei Endpositionen definieren können, nämlich
        – eine sogenannte "vordere Stellung", in welcher die Hülle (12) die Nadel (15) und den Ansatz (14) vollständig überdeckt, sowie
        – eine sogenannte "hintere Stellung", in der mindestens der aktive Teil der Nadel (15) freiliegt und der hintere Verriegelungsabschnitt (22) der Hülle (12) die rohrförmige Hülse (16) und die genannte Muffe (4), welche ineinander geschoben sind, überdeckt, um so die Verbindung zwischen dem Spritzenkörper einerseits und dem Spritzengriff andererseits zu verriegeln.

2. Spritze nach Anspruch 1, dadurch gekennzeichnet, daß die Einsetzvorrichtungen der Hülse (16) des Spritzenkörpers (11) und des Griffs (1) der Spritze aus einer Ringkehle (19) und einer Ringrippe (5) entsprechender Gestalt bestehen, welche derart ausgebildet sind, daß sie zusammenarbeiten, wenn Hülse und Muffe zusammengesetzt sind.

3. Spritze nach Anspruch 2, dadurch gekennzeichnet, daß
    – die Muffe (4) des Spritzengriffes (1) so gestaltet ist, daß sie in die Hülse (16) des Spritzenkörpers (11) eindringen kann,
    – die Ringrippe (5) auf der Muffe (4)) des Spritzengriffes (1) zum Ende der Muffe hin angebracht ist,
    – die Ringkehle (19) in der Hülse (16) des Spritzenkörpers (11) mit Abstand zum Ende des letzteren (11) derart angebracht ist, daß man eine eingesetzte Stellung erhält, in welcher die Muffe (4) des Griffes (1) in der Hülse (16) des Spritzenkörpers (11) steckt.

4. Spritze nach einem der Ansprüche 2 oder 3, dadurch gekennzeichnet, daß die Hülse (16) des Spritzenkörpers (11) mehrere Längsschlitze (17) aufweist, welche sich von ihrem Ende (16a) her erstrecken, um eine radiale Verformung dieser Hülse zu gestatten.

5. Spritze nach einem der vorherigen Ansprüche, dadurch gekennzeichnet, daß die Anschlagmittel aus zwei in den Spritzenkörper (11) eingebrachten äußeren Ringkehlen (21, 18), "vordere und hintere Anschlag-Ringkehle" genannt, sowie aus einer inneren, in der Schutzhülle (12) angebrachten Rippe (23) bestehen.

6. Spritze nach Anspruch 5, dadurch gekennzeichnet, daß
   – die Ringrippe (23) der Schutzhülle (12) zum Ende ihres Verriegelungsabschnittes (22) hin angebracht ist,
   – die hintere Anschlag-Kehle (18) zum Ende der Hülse (16) des Spritzenkörpers (11) hin angebracht ist, um eine hintere Anschlagstellung der Schutzhülle (12) zu erhalten, in welcher diese die rohrförmige Hülse (16) und die Muffe (4) in Einsetzstellung überdeckt,
   – und die vordere Anschlag-Kehle (21) auf dem Spritzenkörper (11) in einem derartigen Abstand vom Ansatz (14) angebracht ist, daß man eine vordere Anschlagstellung der Schutzhülle (12) erhält, in welcher diese die Nadel (15) und den Ansatz (14) vollständig überdeckt.

7. Spritze nach einem der Ansprüche 5 oder 6, dadurch gekennzeichnet, daß die Schutzhülle (12) einen Verriegelungsabschnitt (22) aufweist, der mit mehreren Längsschlitzen (24) versehen ist, die von seinem Ende ausgehen, um seine radiale Verformung zu gestatten.

8. Spritze nach Anspruch 6, dadurch gekennzeichnet, daß der Spritzenkörper (11) zwischen dem Ansatz (14) und der vorderen Anschlag-Kehle (21) eine ringförmige Blockier-Kehle (20) aufweist, welche einen rechteckigen Querschnitt aufweist, der dem der Rippe (23) der Schutzhülle (12) entspricht und so beschaffen ist, daß die Schutzhülle nicht verschoben werden kann, wenn die Rippe (23) in der Blockier-Kehle (20) liegt.

9. Spritze nach Anspruch 3, dadurch gekennzeichnet, daß sie eine innere Rippe (13) aufweist, die
   – in der rohrförmigen Hülse (16) des Spritzenkörpers (11) angebracht ist, und zwar zwischen dem Ansatz (14) und der Ringkehle (19) der Hülse, und
   – eine asymmetrische Form aufweist, um eine Kartusche (26) in den Spritzenkörper (11) einführen zu können und deren Herausziehen zu verhindern.

10. Spritze nach Anspruch 9, dadurch gekennzeichnet, daß sie einen Spritzenkörper (11) und eine Schutzhülle (12) aufweist, die aus durchsichtigem plastikmaterial hergestellt sind, um das Flüssigkeitsniveau in der Kartusche (26) sehen zu können.

11. Spritze nach einem der vorherigen Ansprüche, dadurch gekennzeichnet, daß sie einen Ansatz (14) aufweist, der auf dem Ende des Spritzenkörpers (11) dicht angebracht ist.

12. Spritze nach einem der Ansprüche 1 bis 10, dadurch gekennzeichnet, daß der Spritzenkörper an einem Ende mit einem Gewinde versehen ist, um das Aufschrauben eines mit einer Gewindebohrung versehenen Ansatzes zu gestatten.

13. Für Einmalgebrauch vorgesehene Injektionseinheit für eine Spritze nach einem der Ansprüche 1 bis 12, dadurch gekennzeichnet, daß sie
   a) einen Spritzenkörper, der an seinem einem Ende mit einem Ansatz mit Injektionsnadel versehen ist, um einen aktiven Injektionsteil zu definieren, und an seinem anderen Ende eine rohrförmige Hülse (16) mit Einsetzvorrichtung (19) aufweist,
   b) eine Schutzhülle (12), deren Durchmesser es gestattet, daß sie außen auf dem Spritzenkörper gleitet, mit einem Verriegelungsabschnitt (22), der die rohrförmige Hülse (16) des Spritzenkörpers überdecken kann, sowie
   c) Anschlagmittel, welche auf der Schutzhülle (12) und dem Spritzenkörper (11) angebracht sind und zwei extreme Positionen definieren, nämlich
   – eine sogenannte "vordere Stellung", in welcher die Hülle (12) die Nadel (15) und den Ansatz (14) vollständig überdeckt, sowie
   – eine sogenannte "hintere Stellung", in welcher wenigstens der aktive Teil der Nadel (15) freiliegt und der Verriegelungsabschnitt (22) der Hülle (12) die rohrförmige Hülse (16) des Spritzenkörpers (11) überdeckt, aufweist.

14. Injektionseinheit nach Anspruch 13, dadurch gekennzeichnet, daß die Einsetzvorrichtung der rohrförmigen Hülse (16) des Spritzenkörpers (11) aus einer Ringkehle (19) besteht.

15. Injektionseinheit nach einem der Ansprüche 13 oder 14, dadurch gekennzeichnet, daß die rohrförmige Hülse (16) des Spritzenkörpers (11) mehrere Längsschlitze (17) aufweist, welche von ihrem Ende ausgehen.

16. Injektionseinheit nach einem der Ansprüche 13 bis 15, dadurch gekennzeichnet, daß die Anschlagmittel aus zwei in den Spritzenkörper (11) eingebrachten äußeren Ringkehlen (21, 18), "vordere und hintere Anschlagringkehle"- genannt, sowie aus einer inneren, in die Schutzhülle (12) eingebrachten Ringrippe (23) bestehen.

17. Injektionseinheit nach Anspruch 16, dadurch gekennzeichnet, daß
a) die Ringrippe (23) der Schutzhülle (12) zum Ende ihres Verriegelungsabschnittes (22) hin angebracht ist,
b) die hintere Ringkehle (18) zum Ende der Hülse (16) des Spritzenkörpers (11) hin angebracht ist, um eine hintere Anschlagstellung der Schutzhülle (12) zu erhalten, in welcher diese die rohrförmige Hülse (16) übergreift,
c) die vordere Anschlagkehle (21) auf dem Spritzenkörper (11) in einem derartigen Abstand vom Ansatz (14) angebracht ist, daß man eine vordere Anschlagstellung der Schutzhülle (12) erhält, in welcher diese die Nadel (15) und den Ansatz (14) vollständig überdeckt.

18. Injektionseinheit nach einem der Ansprüche 16 oder 17, dadurch gekennzeichnet, daß die Schutzhülle (12) einen Verriegelungsabschnitt (22) aufweist, der mit mehreren Längsschlitzen (24) versehen ist, die von seinem Ende ausgehen, um seine radiale Verformung zu gestatten.

19. Injektionseinheit nach Anspruch 17, dadurch gekennzeichnet, daß der Spritzenkörper (11) zwischen dem Ansatz und der vorderen Anschlag-Ringkehle (21) eine ringförmige Blockierkehle (20) mit rechteckigem Querschnitt aufweist, der dem der Rippe (23) der Schutzhülle (12) entspricht und so beschaffen ist, daß die Schutzhülle nicht verschoben werden kann, wenn die Rippe (23) in der Blockierkehle (20) liegt.

20. Injektionseinheit nach Anspruch 14, dadurch gekennzeichnet, daß der Spritzenkörper (11) eine innere Rippe (13) aufweist,
– die in seiner rohrförmigen Hülse (16) angebracht ist, und zwar zwischen dem Ansatz (14) und der Ringkehle (19) der Hülse,
– wobei diese Rippe eine asymmetrische Form aufweist, um eine Kartusche (26) in den Spritzenkörper einführen zu können und deren Herausziehen zu verhindern.

21. Injektionseinheit nach Anspruch 20, dadurch gekennzeichnet, daß sie einen Spritzenkörper (11) und eine Schutzhülle (12) aufweist, die aus transparentem plastikmaterial hergestellt sind, um das Flüssigkeitsniveau in der Kartusche (26) sehen zu können.

22. Injektionseinheit nach einem der Ansprüche 13 bis 21, dadurch gekennzeichnet, daß sie einen auf dem Ende des Spritzenkörpers (11) dicht angebrachten Ansatz (14) aufweist.

23. Injektionseinheit nach einem der Ansprüche 13 bis 21, dadurch gekennzeichnet, daß der Spritzenkörper an einem Ende mit einem Gewinde versehen ist, um einen mit einer Gewindebohrung versehenen Ansatz aufschrauben zu können.

24. Verbrauchbares Modul für Einmalgebrauch für zahnärztliche Zwecke, dadurch gekennzeichnet, daß es
a) eine Injektionseinheit nach den Ansprüchen 13 bis 23 aufweist, mit einem Spritzenkörper (11) und einer Schutzhülle (12), die in ihrer vorderen Stellung angeordnet ist, wo sie die Nadel (15) und den Ansatz (14) überdeckt,
b) eine Kartusche (26) aufweist, die im Spritzenkörper (11) angeordnet ist, sowie
c) eine sterile Tasche (25), zum Verpacken der Injektionseinheit, aufweist.

25. Modul nach Anspruch 24, dadurch gekennzeichnet, daß es eine Injektionseinheit nach Anspruch (20) aufweist.

26. Handgriff für eine Spritze gemäß einem der Ansprüche 1 bis 12, dadurch gekennzeichnet, daß er
a) einen Handgriffteil (2, 3) aufweist, der eine Muffe (4) mit einer Einsetzvorrichtung (5) aufweist,
b) einen Kolben (6) aufweist, der im Inneren des Stützteiles (2, 3) und der Muffe (4) beweglich ist.

27. Handgriff nach Anspruch 26, dadurch gekennzeichnet, daß die Einsetzvorrichtung der Muffe (4) aus einer Ringrippe (5) besteht, die auf dem Niveau des Endes der Muffe angebracht ist.

## Claims

1. Syringe comprising a single-use injection needle (15); in particular for dental applications and comprising:
– a syringe grip (1) comprising a piston (6) and a support member (2, 3) through which said piston passes and is able to move along the latter,
– a syringe body (11) provided towards one of its ends with a base (14) supporting the injection needle (15) in order to define an active injection part,
– means (4, 5, 16, 19) for the detachable fixing of the syringe body (11) to the syringe grip (1),
– and a protective case (12) whereof the shape is suitable for housing the needle (15) and its base (14), said syringe being characterised in that:
– the means for the detachable fixing of the syringe body to the grip comprise:
– a tubular end-piece (16) supported by the syringe body (11) remote from the base (14) and having an interlocking structure (19),
– a sleeve (4) integral with the support member (2, 3) of the grip (1) such that the piston (6) passes therethrough and which sleeve has an interlocking structure (5) interacting with that of the end-piece (16) of the syringe body (11), said tubular end-piece (16) and sleeve (4) being of shapes suitable for penetrating one in the other in order to come into an interlocked position where their interlocking structures (5, 19) cooperate,
– the protective case (12) has a diameter adapted to be able to slide externally along the syringe body (11), with a locking portion (22) able to cover the tubular end-piece (16) and said sleeve (4) and to lock them in their interlocked position,
– the protective case (12) and the syringe body (11) comprise abutment means (18, 21, 23) adapted to define two extreme positions: a so called front position where the case (12) completely covers the needle (15) and the base (14), and a so called rear position where at least the active part of the needle (15) is exposed and where the locking portion (22) of the case (12) covers the interlocked tubular end-piece (16) and said sleeve (4), in order to ensure locking of the connection of the syringe body/syringe grip.

2. Syringe according to Claim 1, characterised in that the interlocking structures of the end-piece (16) and of the sleeve (4) of the body (11) and of the syringe grip (1) are constituted by an annular rib (5) and an annular groove (19) of interacting shapes, provided in order to cooperate in the interlocked position of said end-piece and sleeve.

3. Syringe according to Claim 2, characterised in that the sleeve (4) of the syringe grip (1) has a shape adapted for penetrating the end-piece (16) of the syringe body (11),
– the annular rib (5) is provided on the sleeve (4) of the syringe grip (1), towards the end of the latter,
– the annular groove (19) is provided inside the end-piece (16) of the syringe body (11), at a distance from the end of the latter, in order to obtain an interlocked position in which the sleeve (4) of the grip (1) is housed in the end-piece (16) of the syringe body (11).

4. Syringe according to one of Claims 2 or 3, characterised in that the end-piece (16) of the syringe body (11) comprises a plurality of longitudinal slits (17) extending from its end (16a) in order to allow a radial deformation of said end-piece.

5. Syringe according to one of the preceding Claims, characterised in that the abutment means of the protective case (12) are constituted by two outer, annular, so called front and rear abutment grooves (21, 18) provided on the syringe body (11) and by an inner, annular rib (23) provided in the protective case (12).

6. Syringe according to Claim 5, characterised in that the annular rib (23) of the protective case (12) is provided towards the end of the locking portion (22) of the latter,
– the rear abutment groove (18) is provided towards the end of the end-piece (16) of the syringe body (11) in order to obtain a rear abutment position of the protective case (12) in which the latter covers the interlocked tubular end-piece (76) and the sleeve (4),
– and the front abutment groove (21) is provided on the syringe body (11) at a distance from the base (14) adapted to obtain a front abutment position of the protective case (12) in which the latter completely covers the needle (15) and said base (14).

7. Syringe according to one of Claims 5 or 6, characterised in that the protective case (12) comprises a locking portion (22) having a plurality of longitudinal slits (24) extending from its end in order to allow a radial deformation of said portion.

8. Syringe according to Claim 6, characterised in that it comprises an annular locking groove (20) provided on the syringe body (11) between the base (14) and the front abutment groove (21), said locking groove having a rectangular section interacting with that of the rib (23) of the protective case (12) and being adapted to prevent any movement of said case when said rib (23) is housed inside this locking groove (20).

9. Syringe according to Claim 3, characterised in that it comprises an inner rib (13) provided inside the tubular end-piece (16) of the syringe body (11), adjacent the base (14) with respect to the annular groove (19) of said end-piece, said rib having an asymmetrical shape adapted to facilitate the introduction of a cartridge (26) inside the syringe body (11) and to prevent the withdrawal of this cartridge.

10. Syringe according to Claim 9, characterised in that it comprises a syringe body (11) and a protective case (12) made from a transparent plastics material adapted to make it possible to inspect the level of liquid contained in the cartridge (26).

11. Syringe according to one of the preceding Claims, characterised in that it comprises a base (14) sealed on the end of the syringe body (11).

12. Syringe according to one of Claims 1 to 10, characterised in that the syringe body comprises a screw-threaded nose adapted to facilitate the screwing of a base having a tapped bore.

13. Single-use injection arrangement for a syringe according to one of Claims 1 to 12, characterised in that it comprises:
 – a syringe body (11) comprising, towards one of its ends, a base (14) supporting an injection needle (15) in order to define an active injection part, and towards its opposite end, a tubular end-piece (16) comprising an interlocking structure (19),
 – a protective case (12) whereof the diameter is adapted to be able to slide externally along the syringe body (11), with a locking portion (22) able to cover the tubular end-piece (16) of said syringe body,
 – abutment means provided on the protective case (12) and the syringe body (11), adapted to define two extreme positions: a so called front position, where the case (12) completely covers the needle (14) and the base (15), and a so called rear position, where at least the active part of the needle (15) is exposed and where the locking portion (22) of the case (12) covers the tubular end-piece (16) of the syringe body (11).

14. Injection arrangement according to Claim 13, characterised in that the interlocking structure of the tubular end-piece (16) of the syringe body (11) is constituted by an annular groove (19).

15. Injection arrangement according to one of Claims 13 or 14, characterised in that the tubular end-piece (16) of the syringe body (11) comprises a plurality of longitudinal slits (11) extending from its end.

16. Injection arrangement according to one of Claims 13 to 15, characterised in that the abutment means of the protective case (12) are constituted by two external annular grooves (21, 18) so called front and rear abutment grooves, provided on the syringe body (11) and by an inner annular rib (23) provided in the protective case (12).

17. Injection arrangement according to Claim 76, characterised in that:
 – the annular rib (23) of the protective case (12) is provided towards the end of the locking portion (22) of the latter,
 – the rear abutment groove (18) is provided towards the end of the end-piece (16) of the syringe body (11) in order to obtain a rear abutment position of the protective case (12) in which the latter covers the tubular end-piece (16) of the syringe body (11),
 – and the front abutment groove (21) is provided on the syringe body (11) at a distance from the base (14) adapted to obtain a front abutment position of the protective case (12) in which the latter completely covers the needle (15) and said base (14).

18. Injection arrangement according to one of Claims 16 or 17, characterised in that the protective case (12) comprises a locking portion (22) having a plurality of longitudinal slits (24) extending from its end in order to facilitate a radial deformation of said portion.

19. Injection arrangement according to Claim 17, characterised in that the syringe body (11) comprises an annular locking groove (20) provided between the base (14) and the front abutment groove (21), said locking groove having a rectangular section interacting with that of the rib (23) of the protective case (12) and being adapted to prevent any movement of said case when said rib (23) is housed inside this locking groove (20).

20. Injection arrangement according to Claim 14, characterised in that the syringe body (11) comprises an inner rib (13) provided inside the tubular end-piece (16), adjacent the base (14) with respect to the annular groove (19) of said end-piece, said rib having an asymmetrical shape adapted to facilitate the introduction of a cartridge (26) inside the syringe body (11) and to prevent the with-drawal of this cartridge.

21. Injection arrangement according to Claim 20, characterised in that it comprises a syringe body (11) and a protective case (12) made from a transparent plastics arterial adapted to make it possible to inspect the level of liquid contained in the cartridge (26).

22. Injection arrangement according to one of Claims 13 to 21, characterised in that it comprises a base (14) sealed on the end of the syringe body (11).

23. Injection arrangement according to one of Claims 13 to 21, in which the syringe body comprises a screw-threaded nose able to facilitate the screwing of a base having a tapped bore.

24. Single-use consumable module, for dental applications, characterised in that it comprises in combination:
    – an injection arrangement according to one of Claims 13 to 23, comprising a syringe body (11) and a protective case (12) disposed in its front position where it covers the needle (15) and the base (14),
    – a cartridge (26) integrated inside the syringe bly (11),
    – a sterilised pouch (25) for packaging said injection arrangement.

25. Consumable module according to Claim 24, characterised in that it comprises an injection arrangement according to Claim 20.

26. Grip for a syringe according to one of Claims 1 to 12, characterised in that it comprises:
    – a digital support member (2, 3) comprising a sleeve (4) having an interlocking structure (5),
    – a piston (6) able to move inside the support member (2, 3) and the sleeve (4).

27. Grip according to Claim 26, characterised in that the interlocking structure of the sleeve (4) is constituted by an annular rib (5) provided at the level of the end of said sleeve.

Fig 1

Fig 1A

Fig 1B

Fig 1c

Fig 2

Fig 2a

Fig 2B

EP 0 393 166 B1

Fig 3

Fig 4

Fig 5

16

Fig. 6

Fig 7

Fig 8

Fig. 9

Fig. 10

Fig. 11

Fig.12